# EUROPEAN PATENT APPLICATION

(11) **EP 3 607 991 A1**
(43) Date of publication of application: **12.02.2020**
(21) Application number: 17903960.7
(22) Date of filing: 19.04.2017
(51) Int. Cl.: A61M 31/00, A61M 37/00

(54) **TIME REVERSAL TECHNIQUE-BASED SYSTEM AND METHOD FOR MANIPULATING PARTICLES**

(30) Priority: 01.04.2017 CN 201710214161
(71) Applicant: Shenzhen Institutes of Advanced Technology, Shenzhen, Guangdong 518055 (CN)
(72) Inventor: CAI, Feiyan, Shenzhen Guangdong 518055 (CN); LI, fei, Shenzhen Guangdong 518055 (CN); WANG, Chen, Shenzhen Guangdong 518055 (CN); QIU, Weibao, Shenzhen Guangdong 518055 (CN); MENG, Long, Shenzhen Guangdong 518055 (CN); WANG, Congzhi, Shenzhen Guangdong 518055 (CN); XIAO, Yang, Shenzhen Guangdong 518055 (CN); ZHENG, Hairong, Shenzhen Guangdong 518055 (CN)
(74) Representative: Cabinet Beaumont
(86) International application number: PCT/CN2017/081071
(87) International publication number: WO 2018/176526

(57) **Abstract**

The present disclosure relates to the acoustic manipulation technology and, in particular, to a system and method for manipulating particles based on a time reversal technique. An objective of the present disclosure is to manipulate the particles in any path in a non-uniform medium. To achieve the objective, the present disclosure provides the system for manipulating the particles based on a time reversal technique including array transducers, a signal reception and transmission control device and a host. The array transducers are communicatively connected to the signal reception and transmission control device. The signal reception and transmission control device is communicatively connected to the host. Therefore, the present disclosure can be applied to fields such as in vivo site-specific medicament administration and reproductive medicine, and has high practical values. The present disclosure further provides the method for manipulating the particles based on a time reversal technique.

## Description

### TECHNICAL FIELD

The present disclosure generally relates to the acoustic manipulation technology and, in particular, to a system and method for manipulating particles based on a time reversal technique.

### BACKGROUND

As a mechanical wave, a sound wave can exchange momentum and energy with particles in a sound field to generate an acoustic radiation force to manipulate the movements of the particles. The acoustic manipulation technology has been applied into many fields such as physics, chemistry and biology, and has received extensive attention due to the advantages of no contact, no damage and good biocompatibility. The current acoustic manipulation technology and devices are mainly used to manipulate objects in the uniform medium (such as water and air) with uniform acoustic parameters such as a density and a sound velocity because the propagation and distribution of the sound waves are precisely controllable in the uniform medium and the necessary sound field profile can be synthesized. The non-uniform distribution of the acoustic parameters such as the density and the sound velocity in the complex non-uniform medium (such as a human body) results in complex acoustic reflection, scattering, refraction and absorption processes, and it is difficult to accurately model the propagation and energy distribution of the sound waves. Therefore, it is difficult to generate the sound field such as a focusing field, a standing wave field and a vortex field in the non-uniform medium by utilizing the current acoustic manipulation technology and thus it is difficult to manipulate the particles in the non-uniform medium. This greatly limits the applications of the acoustic manipulation devices in the fields such as in vivo site-specific medicament administration and reproductive medicine.

It is known that a pair of ultrasonic transducers has been utilized to generate standing waves (see Wu, J, Acoutiscal tweezers. J. Acoust. Soc. Am. 1991. 89 (5), 2140-2143.) or a single-element high-frequency transducer is utilized to generate the focusing sound field (see Lee J, Teh S, Y Lee, A Kim, H. H Lee, Shung K. K. Single beam acoustic trapping. Appl Phys Lett. 2009. 95 (7), 73701(1)-73707(3).) to manipulate the particles in the water. In addition, two-dimensional array transducers have been developed to precisely control phases in the air or water to achieve of manipulating the particles in any preset path (see Courtney, Charles R. P. Demore, Christine E. M., Wu Hongxiao, Grinenko Alon, Wilcox Paul D., Cochran Sandy, Drinkwater Bruce W. Independent trapping and manipulation of microparticles using dexterous acoustic tweezers; Appl Phys Lett. 2014. 104 (15), 154103(1)-154103(4). or Marzo Asier, Seah Sue Ann, Drinkwater Bruce W., Sahoo Deepak Ranjan, Long Benjamin, Subramanian Sriram; Nature Communications. 2015.6, 1-6. or Yoichi Ochiai, Takayuki Hoshi, Jun Rekimoto. Three-Dimensional Mid-Air Acoustic Manipulation by Ultrasonic Phased Arrays. PLOS one, 9(5), 1-5.). The systems for manipulating the particles involved in the existing art still manipulate the particles in the uniform medium although the systems have some progress compared with those in the previous art. In is still difficult to manipulate the particles in the non-uniform medium. This is because the complex scattering and refraction of the sound waves in the non-uniform medium makes it difficult for a transducer to directly emit the sound field and directly generate the focusing field, the vortex field etc. at a specific site so that it is difficult to generate an acoustic potential well for manipulating the particles. This greatly limits the applications of the acoustic manipulation technology.

In view of this, a new technology needs to be developed to overcome these defects.

### SUMMARY

In view of the defects in the existing art, the present disclosure provides a system and method for manipulating particles based on a time reversal technique to manipulate the particles in any path in a non-uniform medium.

In an aspect, the present disclosure provides a system for manipulating the particles based on a time reversal technique. The system includes array transducers, a point source, a signal reception and transmission control device and a host. The array transducers consist of a single element or multiple elements. The array transducers are communicatively connected to the signal reception and transmission control device. The signal reception and transmission control device is communicatively connected to the host. The host is capable of performing time reversal processing on received signals (preferable acoustic signals). In one embodiment, the present disclosure may further simulate transmission of signals from a virtual point source in a total space (uniform or non-uniform medium) by use of a numerical computation method (such as a finite difference time domain (FDTD) method), perform time reversal processing (perform appropriate signal processing) on the acoustic signals received by virtual probe points (probe positions of the array transducers), and output the processed signals to the array transducers for transmission. This method is particularly applied to in vivo site-specific medicament administration with ultrasonic waves in a case where a point source cannot be arranged in a medium.

Preferably, the array transducers are configured to receive and transmit signals; the signal reception and transmission control device is configured to process and transfer signals received by the array transducers and signals transmitted by the host; and the host is configured to process signals transmitted from the signal reception and transmission control device and transmit signals.

More preferably, the array transducers are configured to receive and transmit the acoustic signals; the signal reception and transmission control device is configured to process and transfer the acoustic signals received by the array transducers and instruction signals transmitted by the host; and the host is configured to process control signals transmitted from the signal reception and transmission control device and transmit the instruction signals.

Preferably, the array transducers are a one-dimensional array or a two-dimensional array, and are composed of at least one row. Four rows of transducers are proved and selected here. In addition, each row of transducer arrays should have a larger number of lattices. Here a lattice transducer with a 22^{∗}22 array is used. The array transducers have a shape of a plane or a circular arc.

The present disclosure further provides a method for manipulating the particles based on a time reversal technique. The method may include the following steps: a point source in a medium moves along any preset path and transmits acoustic signals; array transducers receive the acoustic signals transmitted by the point source and penetrating through a non-uniform medium and sequentially transmit the acoustic signals to a signal reception and transmission control device and a host; the host performs time reversal processing on received acoustic signals; the array transducers are caused to transmit the processed acoustic signals; and the particles move along a preset path (a path which the point source moves through).

Furthermore, the method for manipulating the particles based on the time reversal technique may include the following steps: a host receives acoustic signals from a point source; the host performs time reversal processing on the received acoustic signals and transmits the acoustic signals subject to the time reversal processing to array transducers; the array transducers are caused to transmit the acoustic signals subject to the time reversal processing into a medium; and the particles at a position of the point source (preferably an initial position of the point source) are caused to move along a motion trajectory of the point source. The point source may be a physical point source, and the method may accordingly include the following steps: the physical point source in the medium is caused to move along a path which the particles are to move through and transmit the acoustic signals; the array transducers receive the acoustic signals transmitted by the physical point source in motion and penetrating through a non-uniform medium and sequentially transmit the acoustic signals to a signal reception and transmission control device and the host; the host performs the time reversal processing on the received acoustic signals and transmits the acoustic signals subject to the time reversal processing to the array transducers; the array transducers are caused to transmit the acoustic signals subject to the time reversal processing into the medium; and the particles at a position of the physical point source (preferably an initial position of the physical point source) are caused to move along a motion trajectory of the physical point source. The point source may also be a virtual point source, and the method may accordingly include the following steps: by utilizing a numerical computation method, motion of the virtual point source in a total space with the uniform or non-uniform medium is simulated and transmission of the acoustic signals by the virtual point source is simulated; reception of the acoustic signals by the array transducers and input of the acoustic signals into the host by the array transducers are simulated; the host performs the time reversal processing on the received acoustic signals; the array transducers are caused to transmit the processed acoustic signals; and the particles at a position of the virtual point source (preferably an initial position of the virtual point source) are caused to move along a motion trajectory of the virtual point source.

Preferably, in the method, the time reversal processing refers to that one acoustic signal that is received earlier than another signal is to be transmitted later than the another signal, while one acoustic signal that is received later than another signal is to be transmitted earlier than the another signal.

Preferably, in the method, the particles are captured and manipulated when the acoustic signals transmitted by the array transducers focus at the position of the point source and generate an acoustic potential well.

Preferably, in the method, the point source is continuously moving and the medium is a uniform medium or the non-uniform medium. The method may further include manipulating the motion path of the particles in the medium or manipulating the particles in any path in the medium. Preferably, in the method, a resonance frequency of the array transducers ranges between 1 kHz and 500 MHz, and preferably ranges between 20 kHz and 50 MHz.

Preferably, in the method, the particles are foam balls and the resonance frequency of the array transducers is 40 kHz.

An acoustic time reversal technique is an adaptive focusing method with unique advantages. Regardless of conditions of the medium and the array transducers, the adaptive focusing may be implemented without prior knowledge (such as the spatial distribution of medium parameters such as a density, a sound velocity, an attenuation coefficient and a transducer transmission function). The particles will be captured near the acoustic potential well for manipulating the particles generated at the focusing point. Therefore, the acoustic time reversal technique can implement focusing and particle manipulation in the non-uniform medium. The present disclosure using the acoustic time reversal technique overcomes the current limitation that an acoustic manipulation device cannot manipulate the particles in any path in the non-uniform medium. When the acoustic time reversal technique mentioned in the patent is combined with an electronic system, the particles can be manipulated in any path in any medium (including the uniform medium or the non-uniform medium). Therefore, the acoustic manipulation device can be applied to fields such as in vivo site-specific medicament administration and reproductive medicine and has high practical values.

### BRIEF DESCRIPTION OF DRAWINGS

FIG. 1 is a schematic diagram illustrating connection between components in a system for manipulating particles based on a time reversal technique.
FIG. 2 is a flowchart of a method for manipulating particles based on the time reversal technique.
FIG. 3 is a schematic diagram of signal reception by a system for manipulating particles based on the time reversal technique.
FIG. 4 is a schematic diagram of signal transmission by a system for manipulating particles based on the time reversal technique.
FIG. 5 is a diagram of an acoustic pressure field at a focus point.
FIG. 6 illustrates experimental devices based on the time reversal technique.
FIG. 7 illustrates experimental results for manipulating particles based on the time reversal technique.

### DETAILED DESCRIPTION

Specific embodiments of the present disclosure will be described below in conjunction with the drawings. In the specific embodiments described hereinafter of the present disclosure, some specific features are described for a better understanding of the present disclosure and all the specific features are not essential features for implementing the present disclosure, which is apparent to those skilled in the art. The specific embodiments described hereinafter of the present disclosure are only exemplary specific embodiments of the present disclosure and not intended to limit the present disclosure. In addition, to avoid a difficult understanding of the present disclosure, some well-known techniques are not described herein.

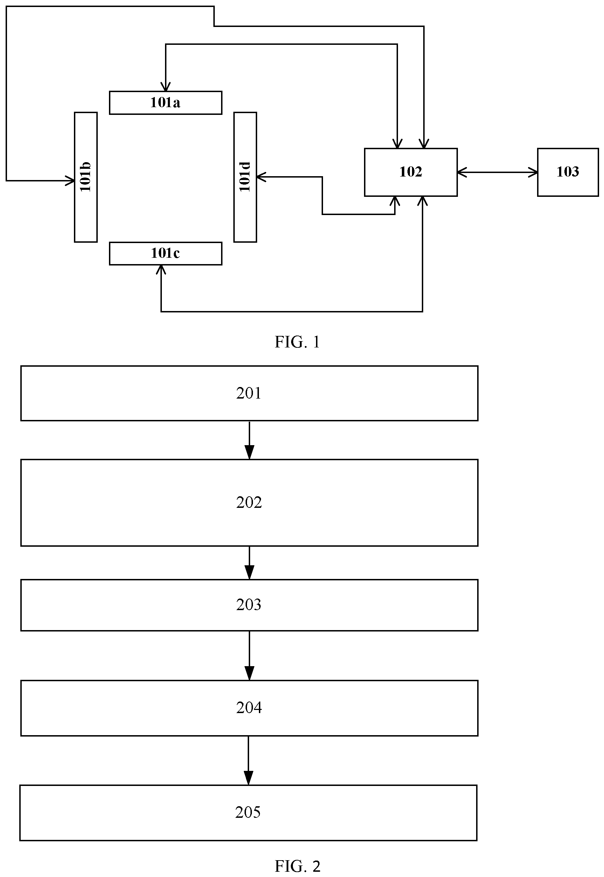

FIG. 1 is a schematic diagram illustrating connection between components in a system for manipulating particles based on a time reversal technique. Reference numerals 101a-101d denote four rows of array transducers arranged in a square, and each row of array transducers includes 22^{∗}22 transducers. A reference numeral 102 denotes a signal reception and transmission control device and a reference numeral 103 denotes a host. The four rows of array transducers are respectively communicatively connected to the signal reception and transmission control device. The signal reception and transmission control device is communicatively connected to the host. The array transducers receive and transmit acoustic signals. The signal reception and transmission control device processes and transfers electronic signals received by the array transducers and transmitted by the host. The host delivers signal instructions and processes control signals transmitted from the signal reception and transmission control device. In another embodiment, there are six rows of array transducers arranged in a hexagon. In another embodiment, there are eight rows of array transducers arranged in an octagon. In another embodiment, the array transducers are arc-shaped and arranged in a circle.

FIG. 2 is a flowchart of a method for manipulating particles based on the time reversal technique. In one embodiment, the method is performed in a non-uniform medium and includes steps 201 to 205. In step 201, a physical point source is arranged in the non-uniform medium to actively transmit the acoustic signals. In step 202, the array transducers receive the acoustic signals transmitted by the physical point source and penetrating through the non-uniform medium, and upload the acoustic signals to a control system and an interface of the host. In step 203, the host performs time reversal processing on the received acoustic signals. In step 204, the array transducers are caused to transmit the acoustic signals subject to the time reversal processing. In step 205, a motion trajectory of the particles at a position of the physical point source is monitored. The non-uniform medium is a medium of different densities (which may be understood as the presence of an obstacle, a human body tissue or a gelatinous liquid, etc., in a manipulation space). The physical point source is a needle transducer smaller than a wavelength, which transmits a sound field which approximates a point source sound field. In another embodiment, the medium is a uniform medium such as air.

In both the above two embodiments, the particles may be captured and manipulated. This is because the acoustic signals transmitted by the planer array transducers focus at the position of a sound source and can generate an acoustic potential well. In the non-uniform medium, the point source is continuously moved in an arbitrary manner, and the array transducers continuously receive the signals and upload all the received signals to the host. The host stores and processes the signals in a way in which one acoustic signal that is received earlier than another signal is to be transmitted later than the another signal, while one acoustic signal that is received later than another signal is to be transmitted earlier than the another signal. In this way, the acoustic signals will also move and focus in the non-uniform medium to generate a moving acoustic potential well. Therefore, the particles can be manipulated in any path.

In another embodiment (not shown in drawings), a virtual point source is used in the method for manipulating particles based on the time reversal technique and the method includes the steps described below. (1) By utilizing a finite difference time domain (FDTD) method, motion of the virtual point source in a total space with the uniform and non-uniform medium is simulated and transmission of the acoustic signals by the virtual point source is simulated. (2) Reception of the acoustic signals by the array transducers and input of the acoustic signals into the host by the array transducers are simulated. (3) The host performs the time reversal processing on the received acoustic signals. (4) The array transducers transmit the processed acoustic signals. (5) The acoustic signals at an initial position of the virtual point source are caused to move along the motion trajectory of the virtual point source. The method described in the embodiment is particularly effective for in vivo site-specific medicament administration with ultrasonic waves in the case where a point source cannot be arranged in the medium, with strong practicability.

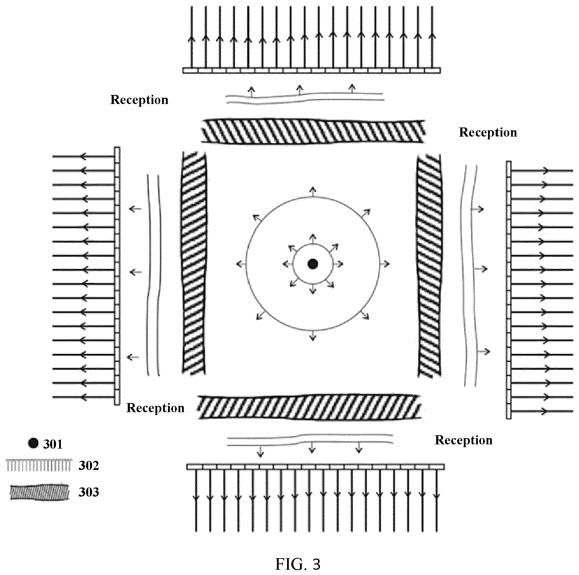

FIG. 3 is a schematic diagram of signal reception by the system for manipulating particles based on the time reversal technique. A reference numeral 301 denotes the point source. A reference numeral 302 is a schematic diagram of a portion taken from the ultrasonic array transducers. A reference numeral 303 is a schematic diagram of a portion of the non-uniform medium (the uniform medium in another embodiment). As shown in FIG. 3, the acoustic signals are transmitted all around from the point source in directions indicated by arrows, penetrate through the non-uniform medium and arrive at the ultrasonic array transducers. The ultrasonic array transducers send the received acoustic signals to the signal reception and transmission control device (not shown in FIG. 3 and referred to 102 in FIG. 1). The signal reception and transmission control device sends the acoustic signals to the host (not shown in FIG. 3 and referred to 103 in FIG. 1).

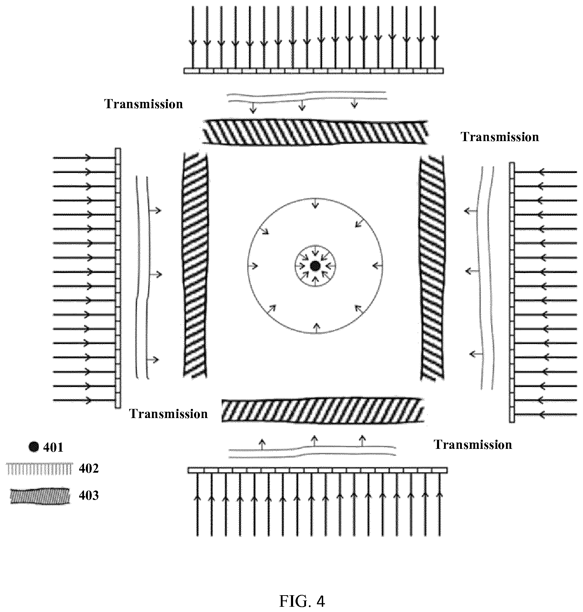

FIG. 4 is a schematic diagram of signal transmission by a system for manipulating particles based on the time reversal technique. A reference numeral 401 denotes the point source. A reference numeral 402 is a schematic diagram of a portion taken from the ultrasonic array transducers. A reference numeral 403 is a schematic diagram of a portion of the non-uniform medium. As described with reference to FIG. 3, the acoustic signals transmitted from the point source pass the non-uniform medium, the ultrasonic array transducers and the signal reception and transmission control device, and arrive at the host. The host performs the time reversal processing on the received acoustic signals, which refers to that one acoustic signal that is received earlier than another signal is to be transmitted later than the another signal, while one acoustic signal that is received later than another signal is to be transmitted earlier than the another signal. The signals are transmitted from the host to acoustic particles in directions opposite to the directions in which the signals are received, that is, the signals pass the signal reception and transmission control device, the ultrasonic array transducers and the non-uniform medium, and arrive at the point source. In this process, since the sound waves emitted by the particles will also move and focus in the non-uniform medium (or the uniform medium), the particles can be manipulated in any path and any medium (including the non-uniform medium and the uniform medium) by combining the system with an electronic system (not shown).

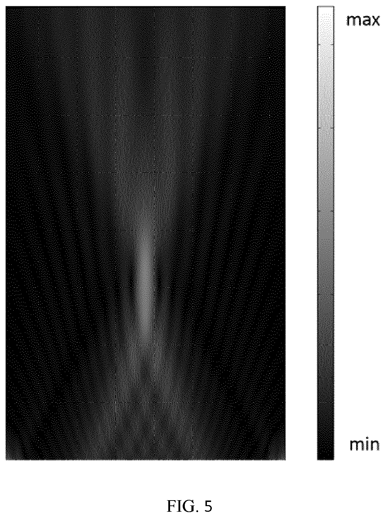

FIG. 5 is a diagram of an acoustic pressure field at a focus point. FIG. 5 shows a focused sound field obtained through numerical computation and simulation by use of the time reversal technique. Simulation results in FIG. 5 are obtained by use of only a single row of array transducers. The sound field is incoming upward from the bottom and the sound field is surrounded by absorbing boundary conditions. A depth of the color in the figure represents an intensity of the sound field; and the darker the color, the stronger the sound field.

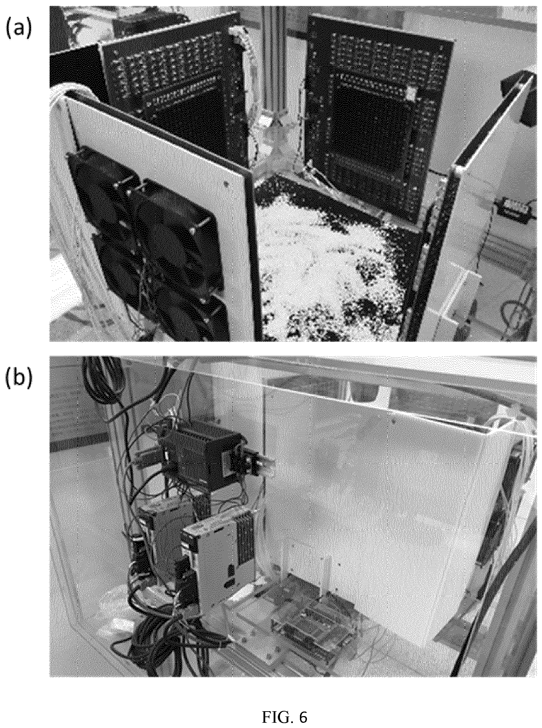

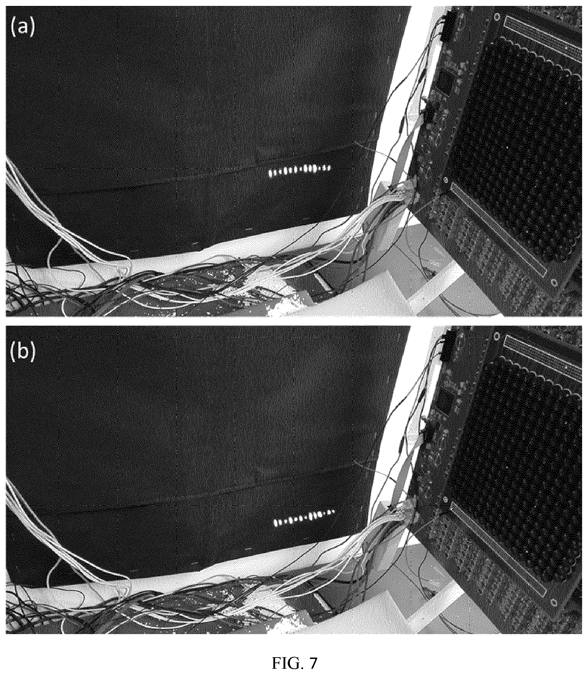

FIG. 6 illustrates experimental devices based on the time reversal technique. FIG. 7 illustrates experimental results for manipulating particles based on the time reversal technique.

Feasibility of the solutions of the present disclosure is proved from theoretical and experimental perspectives in conjunction with the results shown in FIGs 5, 6 and 7. In an experiment, an inventor uses four two-dimensional arrays of 22^{∗}22 transducers with a central frequency of 40 kHz to manipulate the particles. Two ones of the four two-dimensional arrays face to each other, and the other two ones of the four two-dimensional arrays face to each other. FIG. 6(a) shows an experimental device with two-dimensional array transducers. FIG. 6(b) shows a signal reception and transmission control system. A point source is arranged in the manipulation space (including the non-uniform medium) to transmit acoustic signals. After receiving the acoustic signals, the four two-dimensional arrays of transducers process the acoustic signals, perform the time reversal processing on signals received by each transducer, and transmit the processed signals. That means, one acoustic signal that is received earlier than another signal is to be transmitted later than the another signal, while one acoustic signal that is received later than another signal is to be transmitted earlier than the another signal. The acoustic signals will focus at the point source, generate the acoustic potential well, and capture the particles. By adjusting phases, the acoustic signals can focus in any path and generate the acoustic potential well to manipulate the particles in any path. The experimental results are as shown in FIG. 7. FIG. 7 (a) shows captured round foam balls floating in the air at an upper portion with respect to the whole space. FIG. 7(b) shows the captured round foam balls floating in the air at a lower portion with respect to the whole space. Therefore, the experiments show that the present disclosure can manipulate the particles, such as the round foam balls, in any path in the total space.

It is to be noted that the system for manipulating particles based on the time reversal technique refers to any system capable of controlling the reception and transmission of the acoustic signals. A resonant frequency of the transducers used for manipulating the particles is not limited to 40 kHz, and may be any frequency selected for manipulating the particles according to specific conditions and requirements. The manipulated particles are not limited to the foam balls, and may be particles of any diameter, material and shape. The specific number of array elements in the array transducers is not limited. The array transducers may be one-dimensional arrays or two-dimensional arrays. In addition to a plane, the array transducers may have a shape of a circular arc or other appropriate shape.

Although the present disclosure is described through the preferred embodiments, modifications, permutations and various equivalent substitutions are possible within the scope of the present disclosure. It is to be noted that there are many alternative ways of implementing the method and system of the present disclosure. Therefore, it is intended that the appended claims shall be construed to include all the modifications, permutations and various equivalent substitutions within the spirit and scope of the present disclosure.

## Claims

1. A system for manipulating particles based on a time reversal technique, comprising:
array transducers;
a point source;
a signal reception and transmission control device, and
a host for performing time reversal processing on received signals;
wherein the array transducers are communicatively connected to the signal reception and transmission control device; and the signal reception and transmission control device is communicatively connected to the host.

2. The system of claim 1, wherein
the array transducers are configured to receive and transmit signals;
the signal reception and transmission control device is configured to process and transfer signals received by the array transducers and signals transmitted by the host; and
the host is configured to process signals transmitted from the signal reception and transmission control device and transmit signals.

3. The system of claim 2, wherein
the array transducers are configured to receive and transmit acoustic signals;
the signal reception and transmission control device is configured to process and transfer the acoustic signals received by the array transducers and instruction signals transmitted by the host; and
the host is configured to process control signals transmitted from the signal reception and transmission control device and transmit the instruction signals.

4. The system of any one of claims 1 to 3, wherein the array transducers are a one-dimensional array or a two-dimensional array, and comprise at least one row of array elements or more array elements, wherein the array transducers have a shape of a plane or a circular arc.

5. A method for manipulating particles based on a time reversal technique, comprising:
receiving, by a host, acoustic signals from a point source moving in a medium;
performing, by the host, time reversal processing on the received acoustic signals;
transmitting, by the host, the acoustic signals subject to the time reversal processing to array transducers; causing the array transducers to transmit the acoustic signals subject to the time reversal processing into the medium; and
causing the particles in a position of the point source to move along a motion trajectory of the point source.

6. The method of claim 5, wherein the point source is a physical point source and the method further comprises:
causing the physical point source in the medium to move along a path which the particles are to move through and transmit the acoustic signals; and
receiving, by the array transducers, the acoustic signals transmitted from the physical point source in motion and penetrating through a non-uniform medium, and sequentially transmitting the acoustic signals to the signal reception and transmission control device and the host of any one of claims 1 to 4.

7. The method of claim 5, wherein the point source is a virtual point source and the method further comprises:
simulating, by utilizing a numerical computation method, motion of the virtual point source in a total space with a uniform or non-uniform medium and transmission of the acoustic signals by the virtual point source; and
simulating reception of the acoustic signals by the array transducers and input of the acoustic signals into the host by the array transducers.

8. The method of claim 5, 6 or 7, wherein the time reversal processing refers to that one acoustic signal that is received earlier than another signal is to be transmitted later than the another signal, while one acoustic signal that is received later than another signal is to be transmitted earlier than the another signal.

9. The method of claim 5, 6 or 7, further comprising: capturing the particles when the acoustic signals transmitted by the array transducers focus at the position of the point source and generate an acoustic potential well.

10. The method of claim 5, 6 or 7, wherein the array transducers have a resonance frequency between 1 kHz and 500 MHz, and the position of the point source is an initial position of the point source.
